# EUROPÄISCHE PATENTSCHRIFT

(11) **EP 3 676 241 B1**
(45) Veröffentlichungstag und Bekanntmachung des Hinweises auf die Patenterteilung: **26.10.2022**
(21) Anmeldenummer: 18755480.3
(22) Anmeldetag: 20.08.2018
(51) Int. Cl.: C07C 45/75, C07C 47/22, C07C 67/39, C07C 69/54, C08L 33/12, C08F 220/14

(54) **VERFAHREN ZUR HERSTELLUNG OPTISCHER FORMMASSEN**
METHOD FOR PRODUCING OPTICAL MOULDING COMPOSITIONS
PROCÉDÉ DE FABRICATION DES MATIÈRES À MOULER OPTIQUES

(30) Priorität: 29.08.2017 EP 17188252
(43) Veröffentlichungstag der Anmeldung: 08.07.2020
(73) Patentinhaber: Röhm GmbH, 64295 Darmstadt (DE)
(72) Erfinder: KRILL, Steffen, 64367 Mühltal (DE); AIT AISSA, Belaid, 64287 Darmstadt (DE); ZSCHUNKE, Florian, 60433 Frankfurt (DE); KÖLBL, Gerhard, 64579 Gernsheim (DE); CARLOFF, Rüdiger, 64291 Darmstadt (DE)
(74) Vertreter: Röhm Patent Association
(86) Internationale Anmeldenummer: PCT/EP2018/072419
(87) Internationale Veröffentlichungsnummer: WO 2019/042807

(56) Entgegenhaltungen:
- WO-A1-2011/051032
- WO-A1-2014/170223
- WO-A1-2017/046110
- US-B2- 7 012 039

## Beschreibung

### Gebiet der Erfindung

Die vorliegende Erfindung betrifft ein Verfahren zur Herstellung von optischen Formmassen auf Basis von Methylmethacrylat, wobei dieses MMA mittels einer optimierten Methode hergestellt wurde und sich die Formmassen insbesondere durch einen sehr geringen Gelbwert auszeichnen. Das erfindungsgemäß verwendete MMA wurde dabei durch direkte oxidative Veresterung von Methacrolein hergestellt.

Insbesondere betrifft die vorliegende Erfindung eine optimierte Aufarbeitung des Reaktoraustrags der oxidativen Veresterung von Methacrolein, mittels der besonders färbende Nebenprodukte entfernt werden. Darüber hinaus hat dieses Verfahren den Vorteil, dass an die Apparative Ausgestaltung der Anlage weniger Ansprüche als im Stand der Technik beschrieben gestellt werden müssen.

### Stand der Technik

Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierenden Spezialester, die durch Umesterung mit dem entsprechenden Alkohol hergestellt werden können. Hieraus ergibt sich ein großes Interesse an möglichst einfachen, wirtschaftlichen und umweltschonenden Herstellungsverfahren für diesen Ausgangsstoff. Dabei ist es besonders von Interesse, MMA oder andere Methacrylatalkylester zur Verfügung zu stellen, die in dem Polymer, z.B. zur Verarbeitung zur Formmasse zu besonders geringen Gelbfärbungen führen.

Methylmethacrylat (MMA) wird heute mittels diverser Verfahren, die von C₂- C₃- oder C₄-Bausteinen ausgehen, hergestellt. In einem als besonders effizient geltenden Verfahren wird MMA durch Oxidation von Isobutylen oder tert-Butanol mit Luftsauerstoff in der Gasphase am heterogenen Kontakt zu Methacrolein und anschließender oxidativer Veresterungsreaktion von Methacrolein unter Verwendung von Methanol erhalten. Dieses von ASAHI entwickelte Verfahren ist unter anderem in den Druckschriften US 5,969,178 und US 7,012,039 beschrieben. Nachteil dieses Verfahrens ist insbesondere ein sehr hoher Energiebedarf. In einer Weiterentwicklung des Verfahrens wird das Methacrolein in der ersten Stufe aus Propanal und Formaldehyd gewonnen. Ein solches Verfahren ist in WO 2014/170223 beschrieben.

In US 5,969,178 ist ein solches Verfahren zur oxidativen Umsetzung von Isobuten oder tert-Butanol zu Methacrolein und die folgende oxidative Veresterung zu MMA beschrieben. In dieser zweiten Stufe wird eine flüssige, im Wassergehalt reduzierte Mischung aus Methacrolein und Methanol mit molekularem Sauerstoff und einem Palladiumkatalysator umgesetzt, wobei dieser zumeist auf einem Träger als Palladium- Blei-Katalysator vorliegt. Aus dem Rohprodukt der oxidativen Veresterung wird darauf in einer ersten Destillationsstufe ein Gemisch aus Methacrolein und Methanol unterhalb des Kopfes der Kolonne abgetrennt, während über Kopf leichtsiedende Bestandteile entfernt werden. Der MMA-haltige Sumpf wird darauf in eine zweite Destillationsstufe geführt, in der über Kopf ein Azeotrop aus Methanol und gesättigten Kohlenwasserstoffen abgetrennt wird. Der Sumpf, enthaltend das Roh-MMA wird einer weiteren Aufarbeitung zugeführt, während aus der über Kopf gewonnen Fraktion mittels einem Phasentrenner und einer dritten Destillationskolonne Methanol isoliert und zurück in den Reaktor geführt wird. Dabei ist zu berücksichtigen, dass das Methanol aufgrund des gebildeten Azeotrops relativ viel Wasser enthalten kann und damit einer Entwässerung zugeführt werden muss.

Als Alternative zu diesem Verfahren offenbart die US 5,969,178 die Aufarbeitung in nur einer Kolonne, wobei sich bei dieser der Zulauf zwingend oberhalb des Sumpfes befindet. Über Kopf werden dieser Kolonne leichtsiedende Bestandteile aus dem Reaktoraustrag entfernt. Im Sumpf verbleibt eine Mischung aus Roh-MMA und Wasser, welche einer weiteren Aufarbeitung zuzuführen ist. Über einen Seitenstrom, dessen genaue Lage zunächst bestimmt werden muss und die durch Hinzufügen verschiedener Siedeböden eingestellt werden kann, wird der Kolonne schließlich eine Mischung aus Methacrolein und Methanol, gedacht zur Zurückführung in den Reaktor, entnommen. US 5,969,178 weist dabei selbst darauf hin, dass ein solches Verfahren aufgrund diverser Atzeotrope schwierig durchzuführen ist. Darüber hinaus spielt dabei insbesondere Methacrylsäure, die immer als Nebenprodukt vorliegt eine große Rolle. Nach diesem Verfahren, auch wenn die US 5,969,178 darüber schweigt, würde die Methacrylsäure derart abgetrennt werden, dass sie in einer der Entsorgung zuzuführenden Phase verbleibt und sich eine Isolierung nur bedingt lohnen würde. Damit jedoch sinkt die Gesamtausbeute an methacrylischen Produkten dieses Verfahrens.

In US 7,012,039 wird eine etwas abweichende Aufarbeitung des Reaktoraustrags der oxidativen Veresterung offenbart. Hier wird in einer ersten Destillationsstufe über Siebböden Methacrolein über Kopf abdestilliert und die wässrige, MMA haltige Mischung aus dem Sumpf in einen Phasenseperator geleitet. In diesem wird die Mischung durch Zugabe von Schwefelsäure auf einen pH-Wert von ca. 2 eingestellt. Die Trennung des schwefelsauren Wassers von der organischen bzw. Öl-Phase erfolgt dann mittels Zentrifugieren. Diese Ölphase wird in einer weiteren Destillation in hochsiedende Bestandteile und eine MMA haltige Phase, welche über Kopf abgezogen wird, getrennt. Die MMA haltige Phase wird danach in einer dritten Destillation von niedrigsiedenden Bestandteilen getrennt. Darauf folgt noch eine vierte Destillation zur endgültigen Reinigung.

Problematisch an diesem Verfahren ist die Schwefelsäure, die in großen Mengen zugegeben werden muss und in Teilen der Anlage korrosiv wirken kann. Entsprechend müssen diese Teile, wie insbesondere der Phasenseperator oder auch die zweite Destillationskolonne aus dafür geeigneten Materialien gefertigt sein.

Weiterhin schweigt auch die US 7,012,039 über den Umgang mit der gleichsam anfallenden Methacrylsäure oder dem im Produkt verbleibenden restlichen Methanol. Es ist jedoch zu vermuten, dass die erstgenannte in den Destillationsstufen mit abgetrennt wird, während das Methanol nur teilweise mit dem Methacrolein gewonnen und zurückgeführt werden kann, während der Rest wahrscheinlich in der dritten Destillationsstufe verloren geht.

WO 2014/170223 beschreibt ein ähnliches Verfahren wie US 7,012,039. Es besteht nur der Unterschied, dass in der eigentlichen Reaktion mittels Zugabe einer methanolischen Natriumhydroxidlösung in einer Kreislaufführung der pH-Wert eingestellt wird. Dies dient unter anderem dazu, den Katalysator zu schonen. Weiterhin ist die Abtrennung der wässrigen Phase in der Phasenseperation aufgrund des Salzgehalts einfacher. Es führt jedoch auch dazu, dass die entstehende Methacrylsäure als Natriumsalz vorliegt und später mit der wässrigen Phase abgetrennt und verworfen wird. Bei der Variante einer Schwefelsäurezugabe in der Phasenseperation wird die freie Säure zwar wiedergewonnen. Dafür fällt jedoch Natrium(hydrogen)sulfat an, welches bei der Entsorgung zu anderen Problemen führen kann.

Die WO 2017/046110 schließlich lehrt ein optimierte Aufarbeitung des aus einer oxidativen Veresterung erhaltenen roh-MMA zunächst von einer Schwerphase getrennt wird und aus dieser Schwerphase anschließend eine Alkohol-enthaltende Leichtphase abdestilliert wird, die wiederum recycelt werden kann. Das Besondere an diesem Verfahren ist zudem, dass das Methacrolein hier auf Basis von Propanal und Formaldehyd, wobei ersteres auf Basis von C2-Bausteinen, z.B. aus Ethylen und Synthesegas, gewonnen wird, erhalten wurde.

Insgesamt führen jedoch alle diese Verfahren, unabhängig von der Rohstoffbasis für das eingesetzte Methacrolein zu MMA oder im Allgemeinen Alkylmethcrylaten, die zu einer Gelbfärbung der Folgeprodukte, wie z.B. Formmassen. Es besteht also ein Verbesserungsbedarf derart, die Quelle dieser Gelbfärbung zu identifizieren und vor der Polymerisation möglichst effizient aus dem entsprechenden Alkylmethacrylat, insbesondere MMA zu entfernen.

### Aufgabe

In Anbetracht des Standes der Technik ist es daher Aufgabe der vorliegenden Erfindung, ein technisch verbessertes Verfahren zur Herstellung von Polymerharzen zur Verfügung zu stellen, die eine besonders geringe Gelbfärbung aufweisen.

Insbesondere bestand die Aufgabe, die zur Herstellung dieser Polymerharze eingesetzten Alkylmethacrylate, insbesondere MMA auf Basis einer oxidativen Veresterung von Methacrolein zur Verfügung zu stellen.

Darüber hinaus lag der vorliegenden Erfindung die Aufgabe zugrunde, die bei der oxidativen Veresterung von Methacrolein entstehenden, zur Gelbfärbung der Folgeprodukte führenden Nebenprodukte zu identifizieren und effizient aus dem Alkylmethacrylat zu entfernen.

Insbesondere bestand auch die Aufgabe, ein Verfahren zur Verfügung zu stellen, das mit einem möglichst geringen Entsorgungsaufwand, insbesondere durch ein reduziertes Anfallen organischer Bestandteile und Säuren im Abfallstrom, betrieben werden kann.

Weiterhin soll das Verfahren gegenüber dem Stand der Technik kostengünstig, insbesondere in Bezug auf die bei der Konstruktion der Anlage zu verwendenden Materialien sein.

### Lösung

Gelöst wurden diese Aufgaben durch die Entwicklung eines neuartigen Verfahrens zur Herstellung von Alkylmethacrylatharzen. Dieses Verfahren weist die folgenden Verfahrensschritte auf:
a. Herstellung von Methacrolein in einem Reaktor I,
b. oxidative Veresterung des Methacroleins in Anwesenheit eine Alkohols, Sauerstoffs und eines heterogenen Edelmetall-haltigen Katalysators bei einem Wassergehalt zwischen 0,1 und 10 Gew% und einem pH-Wert zwischen 5 und 8, bevorzugt zwischen 6 und 8 in mindestens einem Reaktor II,
c. Nachbearbeitung des Reaktoraustrags aus Reaktor II in einem Reaktor III,
d. Isolierung und Reinigung des roh-Alkylmethacrylats aus Reaktor III und
e. Polymerisation der Alkylmethacrylate aus Verfahrensschritt d oder einer Mischung enthaltend Alkylmethacrylate aus Verfahrensschritt d zum Erhalt eines Alkylmethacrylatharzes.

Dabei gilt es festzustellen, dass die einzelnen Verfahrensschritte nicht kontinuierlich, direkt auf einander folgend durchgeführt werden müssen. Auch können weitere Verfahrensschritte, wie beispielsweise Zwischenreinigungen, zwischen den aufgeführten Schritten a bis e durchgeführt werden. Bevorzugt erfolgen die Verfahrensschritte a bis d, optional ergänzt durch Zwischenschritte, aufeinanderfolgend in der angegebenen Reihenfolge und in einem kontinuierlichen Betrieb. Verfahrensschritt e dagegen kann zeitlich und räumlich deutlich getrennt von den anderen Schritten, nach einer optionalen zusätzlichen Reinigung, Transport und/oder Lagerung auch Wochen oder sogar Monate nach der Monomersynthese der Schritte a bis d durchgeführt werden.

Erfindungsgemäß zeichnet sich die vorliegenden Erfindung insbesondere dadurch aus, dass in Verfahrensschritt c in Reaktor III der Wassergehalt um mindestens 0,5 Gew%, bevorzugt um mindestens 0,75 Gew%, insbesondere bevorzugt um mindestens 1 Gew% höher ist als in Reaktor II. Zusätzlich ist die Alkoholkonzentration in Verfahrensschritt c in Reaktor III geringer ist als in Reaktor II, in dem Verfahrensschritt b durchgeführt wird. Schließlich ist dieses neuartige Verfahren dadurch gekennzeichnet, dass der pH-Wert in Reaktor III zwischen 0,5 und 7, insbesondere zwischen 0,5 und 6 liegt und dabei mindestens 0,5 geringer als in Reaktor II eingestellt wird.

Grundsätzlich kann das Methacrolein in Verfahrensschritt a in Reaktor I auf Basis von C2- oder C4-Bausteinen hergestellt werden. Bevorzugt handelt es bei Verfahrensschritt a um die Umsetzung von Propanal mit Formaldehyd in Gegenwart mindestens einer Säure und optional eines Amins, also um einen Verfahrensschritt, der von C2-Bausteinen ausgeht. Insbesondere kann das erfinderische Verfahren auf die Kombinationen aus einem solchen C2-basierten Verfahren zur Herstellung des Methacroleins und eine anschließende oxidative Veresterung zu einem Alkylmethacrylat in Verfahrensschritt b angewendet werden. Dies betrifft insbesondere die Beschreibungen für eine solche Kombination der Verfahrensschritte a und b, wie sie beispielsweise in DE 3 213 681, US 4,408,079, CN 1 038 461 04 oder in der europäischen Patentanmeldung mit dem Anmeldeaktenzeichen 14185345.7 zu finden ist.

Bevorzugt handelt es sich bei dem Alkohol in Verfahrensschritt b um Methanol und bei dem Alkylmethacrylat, welches sich als Rohprodukt aus diesem Verfahrensschritt ergibt, entsprechend um MMA.

Bevorzugt wird in Verfahrensschritt c dem Reaktor III zur Einstellung des pH-Werts eine organische und/oder eine mineralische Säure zugegeben. Dabei gleichzeitig oder unabhängig und genauso bevorzugt wird in diesem Reaktor III Dimethoxyisobuten mit Wasser in Methacrolein und Methanol gespalten.

Die Säurezugabe kann dabei dergestalt erfolgen, dass diese direkt in Reaktor III geleitet wird. Alternativ kann die Säure jedoch auch der Zuleitung aus z.B. Reaktor II in Reaktor III zugeführt werden. Weiterhin möglich ist, dass das Rohprodukt aus Reaktor II erst in einer Mischkammer mit der Säure versehen wird, bevor eine Zuleitung dieser Mischung in den Reaktor III erfolgt.

Insbesondere bevorzugt ist das erfindungsgemäße Verfahren in Bezug auf Verfahrensschritt c dadurch gekennzeichnet, dass zur Einstellung des pH-Werts Schwefelsäure in Reaktor III gegeben wird, und dass die Flüssigphase in Reaktor III eine Temperatur zwischen 0 und 140 °C aufweist. Dabei hängt diese Innentemperatur, gemessen in der Flüssigphase, insbesondere von der genauen Ausgestaltung des verwendeten Reaktors ab. Erfindungsgemäß gibt es bezüglich der genauen Ausgestaltung des Reaktors III vier besonders bevorzugte Ausführungsformen:
In der ersten Ausführungsform handelt es sich bei Reaktor III um eine Destillationskolonne. In diesem Fall wird die Säure, optional zusammen mit zusätzlichem Wasser, bevorzugt in den Sumpf dieser Destillationskolonne eingeleitet. In diesem Kolonnensumpf finden sich dabei unter anderem flüssiges Methacrolein und Teile des verbleibenden Alkohols, die dabei von dem roh-Alkylmethacrylat bei einer Temperatur zwischen 50 und 100 °C getrennt werden.

In der zweiten bevorzugten Ausführungsform des Verfahrensschritts c handelt es sich bei Reaktor III um einen Phasentrenner, in dessen wässrige Phase die Säure und optional zusätzliches Wasser eingeleitet werden. In diesem Phasentrenner wird eine wässrige Phase, enthaltend den verbleibenden Alkohol, von einer organischen Phase, enthaltend das Alkylmethacrylat, bei einer Temperatur zwischen 0 und 100 °C getrennt.

In der dritten bevorzugten Ausführungsform des Verfahrensschritts c handelt es sich bei Reaktor III um einen Rohrreaktor, in dem der Reaktoraustrag aus Reaktor II mit einer Innentemperatur zwischen 50 und 140 °C, die Säure und optional zusätzliches Wasser vermischt werden. Diese Mischung kann anschließend in eine Destillationskolonne oder einen Phasentrenner geleitet werden.

In der vierten bevorzugten Ausführungsform des Verfahrensschritts c handelt es sich bei Reaktor III um einen kontinuierlich betriebenen Rührreaktor. Auch in diesem Reaktor wird analog zu dem zuvor beschriebenen Rohrreaktor der Reaktoraustrag aus Reaktor II bei einer Innentemperatur zwischen 50 und 140 °C mit der Säure und optional zusätzlichem Wasser vermischt. Anschließend wird diese Mischung bevorzugt in eine Destillationskolonne oder einen Phasentrenner geleitet.

Die genaue Ausgestaltung des Verfahrensschritts d ist dem Fachmann leicht, insbesondere in Hinblick auf die genaue Ausgestaltung der vorangehenden Verfahrensschritte herleitbar. Dabei können eine Reihe verschiedener Reinigungsstufen, bevorzugt, jedoch nicht zwangsläufig in Serie geschaltet, eingesetzt werden. Besonders bevorzugt handelt es sich bei den Isolierungs- und Reinigungsstufen um mindestens einen optionalen Phasentrenner, mindestens eine Hochsiederkolonne, mindestens eine Niedrigsiederkolonne und optional um mindestens eine Kristallisationskammer. Besonders bevorzugt werden diese Vorrichtungen in Reihe durchlaufen.

Einen weiteren wichtigen Verfahrensschritt stellt Verfahrensschritt e, die Polymerisation des Produktes aus Verfahrensschritt d, dar. Bezüglich Verfahrensschritt e gibt es naturgemäß viele alternative Ausführungsformen. So kann in Verfahrensschritt e aus dem Alkylmethacrylat oder aus einer Mischung, enthaltend mindestens ein Alkylmethacrylat aus Verfahrensschritt d ein Polymer oder eine Mischung, enthaltend ein Polymer, hergestellt werden. Bei einer solchen Mischung kann es sich zum Beispiel um eine nur teilweise polymerisierte Monomermischung, z.B. in Form eines Sirups oder eines sogenannten MoPo (Monomer-Polymer-System) handeln. Die Polymerisation kann dabei mittels Substanz-, Emulsions-, Suspensions- oder Lösungspolymerisation erfolgen. In der Regel handelt es sich bei der Polymerisation um eine radikalische Polymerisation. Es ist aber auch möglich andere Polymerisation wie eine anionische oder beispielsweise eine Gruppen-Transfer-Polymerisation einzusetzen. Bei der Polymerisation wird erfindungsgemäß ein Alkylmethacrylatharz hergestellt, welches beispielsweise anschließend zu einer Formmasse oder in Mischung mit anderen Komponenten zu einer Formmasse verarbeitet werden kann.

Polymerharze sind durch Wahl einer geeigneten Temperatur thermoplastisch verformbare Polymere. Je nach Molekulargewicht der Polymerketten des Polymeren, Polymeranteil im Polymerharz und einer optional vorliegenden Teilvernetzung der Polymerketten, kann die Umformtemperatur bei Alkylmethacrylatpolymeren bei über 320°C liegen. Sollte die Polymerisation von Alkylmethacrylaten in Gegenwart von vernetzend wirkenden Monomeren oder Stoffen erfolgen, so steigt in der Regel der Anteil an Polymerketten die miteinander vernetzt sind. Die notwendige Temperatur für die thermoplastische Umformung des Polymerharzes wird dadurch steigen. Bei sehr hohen Gehalten Vernetzermonomeren oder Stoffen, die zur Vernetzung führen steigt die notwendige Umformtemperatur bis in den Bereich der thermischen Degradation des Polymerharzes.

Erfindungsgemäß werden unter einem Polymerharz auch solche, wie bereits ausgeführte Systeme verstanden, die nur teilweise polymerisiert wurden.

Erfindungsgemäß können die zu polymerisierenden Zusammensetzungen neben den obigen, erfindungsgemäß über die Verfahrensschritte a bis d hergestellten Alkylmethacrylaten auch weitere ungesättigte Monomere aufweisen, die mit Methylmethacrylat und den zuvor genannten (Meth)acrylaten copolymerisierbar sind. Hierzu gehören unter anderem Alkyl(meth)acrylate, Methylacrylat, Ethylacrylat, Butylacrylat, Cyclohexyl(meth)acrylat, Norbornyl(meth)acrylat, Styrol, substituierte Styrole, Vinylcyclohexan, Vinylacetat, (Meth)acrylsäure, Glutarsäureanhydrid, Maleinsäureanhydrid, n-lsopropyl(meth)acrylamid, (Meth)acrylamid und Acrylnitril.

Optional enthält das Polymerharz Zusatzstoffe bzw. Additive, die diesem vor oder nach Verfahrensschritt e, bevorzugt vor Verfahrensschritt e zugesetzt werden können. Hierzu gehören unter anderem UV-Stabilisatoren, UV-Absorber, Gleitmittel, Antistatika, Flammenschutzmittel, Additive zur Erhöhung der Kratzfestigkeit, Antioxidantien, Lichtstabilisatoren, organische Phosphorverbindungen, Verwitterungsschutzmittel und/oder Weichmacher.

Insbesondere gibt es erfindungsgemäß bezüglich Verfahrensschritt e drei besonders bevorzugte Ausführungsformen:
In der ersten Ausführungsform, das Verfahren der Suspensionspolymerisation, wird eine thermoplastisch verformbare Formmasse erhalten, in dem das Polymerharz mit hohem Monomerumsatz, z.B größer gleich 80% optional getrocknet wird, um nicht umgesetzten Alkylmethacrylate, andere Monomere oder Wasser abzuziehen. Das erhaltene Perlpolymerisat kann dann optional in einem Entgasungsapparat, wie beispielsweise einem Kneter oder einem Entgasungsextruder, weiter entgast und anschließend granuliert werden. Die Granulate oder Polymerharzperlen können im nachfolgenden Schritt in geeigneten Verarbeitungsapparaten zu den gewünschten Formkörpern weiter verarbeitet werden.

In einer zweiten Ausführungsform, das Verfahren der Lösungs- oder Substanzpolymerisation, wird aus Verfahrensschritt d oder aus einer Mischung enthaltend zumindest ein Alkylmethacrylat gemäß Verfahrensschritt d mittels radikalischer Polymerisation ein Polymersirup gebildet. Dieser Sirup wird darauf optional in einem geeigneten Entgasungsapparat entgast, um nicht umgesetzten Alkylmethacrylate, andere Monomere oder Wasser abzuziehen. Der entgaste Polymersirup wird dann optional granuliert. Die Granulate können im nachfolgenden Schritt in geeigneten Verarbeitungsapparaten zu den gewünschten Formkörpern weiter verarbeitet werden.

In einer dritten Ausführungsform, das Verfahren der Blockpolymerisation, wird die Polymerisation einer optional Lösungsmittel enthaltenen Mischung bis zu einem Feststoffgehalt von 80 Gew% durchgeführt. Bevorzugt wird bei dieser Ausführungsform ein lösungsmittelfreies System bis zu einem Umsatz von kleiner 80% polymerisiert. Bevorzugt wird die Polymerisation in einem lösungsmittelfreien System bis zu einem Umsatz von kleiner 80% durchgeführt. Anschließend wird der Polymersirup dann in eine Form gegossen. Dabei können Zuschlagstoffe zugegeben werden. In der Form wird die Polymerisation des Polymersirups dann zu höheren Umsätzen fortgeführt. Enthält der Polymersirup keine Vernetzermonomere oder ist der Gehalt an Vernetzermonomeren gering, dann kann die Form anschließend thermisch umgeformt werden. Bei höheren Gehalten an Vernetzermonomeren ist eine thermische Umformung deutlich schwieriger.

Optimalerweise werden die zumindest optional in allen drei Ausführungsformen gewonnenen durch Entgasung gewonnenen Recyclate zurückgeführt und in einem weiteren Polymerisationsschritt e eingesetzt. Dabei kann es zur Anreicherung von Nebenprodukten, insbesondere solchen mit geringerem Dampfdruck während des Prozesses kommen. Diese Anreicherung, insbesondere mit den bei dem C2-Verfahren gebildeten Dimethoxyisobuten (DMIB) und Methylisobutyrat führt bei späteren Chargen zu einer weiter zunehmenden Gelbfärbung des Formkörpers. Daher müssen nach einigen Recyclaten Teile des Recyclates verworfen werden, um den Recyclatkreislauf bezüglich dieser Nebenprodukte abzureichern. Dies wiederum führt zu einer Verringerung der Polymergesamtausbeute. Mit dem erfindungsgemäßen Verfahren ist es jetzt überraschend möglich, deutlich mehr Chargen unter Rückführung und Wiederverwendung des Recyclats durchzuführen und somit die Polymergesamtausbeute relevant zu steigern.

Mit dem C2-Verfahren werden erfindungsgemäß Verfahren beschrieben, die bei der Synthese eines Alkylmethacrylats von einem C2-Baustein ausgehen. Besonders bevorzugt wird im Rahmen der vorliegenden Erfindung dazu das Propanal in Verfahrensschritt a) auf Basis von Ethylen und Synthesegas gewonnen.

Die erfindungsgemäß hergestellten Alkylmethacylatharze können vielfältig Verwendung finden. Dabei ist einerseits zwischen transparenten und nicht transparenten und andererseits zwischen farbigen und farblosen Formkörpern, die aus den Alkylmethacylatharzen hergestellt werden, zu unterscheiden.

So können transparente Formkörper, bevorzugt farblose, transparente Alkylmethacylatharze insbesondere zur Herstellung von Formkörpern zur Verwendung als Lichtleiterplatten, als Scheinwerferlinsen, in Scheinwerferabdeckungen, in Abdeckungen von Lichtquellen, in Displayabdeckungen, in Lärmschutzwände oder in der Konstruktion von Gewächshäusern eingesetzt werden. Naturgemäß ist insbesondere für farblose Produkte, ganz besonders für transparente, farblose Produkte der Gelbwert des Polymers, aus dem der Formkörper hergestellt wurde, von besonders hoher Bedeutung, so dass mit dem erfindungsgemäßen Verfahren jetzt überraschend auch Alkylmethacrylate, hergestellt auf Basis von C2-Rohstoffen, eingesetzt werden können.

Farbig eingefärbte Alkylmethacylatharze, insbesondere solche die gefüllt und damit intransparent sind, können zu Formkörpern, bevorzugt zur Verwendung in Abdeckungen, in Säulenblenden oder in Zierleisten im PKW-Innen- und/oder Außenbereich eingesetzt werden. Jedoch selbst für gefärbt, intransparent verwendete Alkylmethacylatharze spielt der Gelbwert in Bezug auf die Farbechtheit, die Farbstabilität und die universelle Verwendbarkeit der Farbrezepturen bei Alkylmethacrylaten aus anderen Verfahren eine große Rolle.

Neben dem aufgeführten Verfahren sind auch neuartige Alkylmethacrylate, welche beispielsweise als Produkt aus den erfindungsgemäßen Verfahrensschritten a bis d gewonnen werden können, beschrieben. Diese sind jedoch nicht Bestandteil der vorliegenden Erfindung. So zeichnen sich diese neuartigen Alkylmethacrylate dadurch aus, dass das Alkylmethacrylat zwingend DMIB als Bestandteil aufweist. Darüber hinaus weist das Alkylmethacrylat in der Regel auch Methylisobutyrat auf.

Insbesondere handelt es sich bei diesen Alkylmethacrylaten um solche, die mittels einem sehr vorteilhaften Verfahren, welches von einer C2- anstelle einer C3- oder C4-Basis als Grundbaustein der Methacrylatsynthese ausgeht, hergestellt werden können. C3- oder C4-basierte Alkylmethacrylate weisen dieses Nebenprodukt in der Regel gar nicht auf. Neu ist dabei insbesondere an diesem Alkylmethacrylat, dass dieses im Vergleich zu den im Stand der Technik beschriebenen Materialien zwar DMIB aufweist, dieses jedoch in einem nicht bekannten Gehalt kleiner 300 ppm, bevorzugt kleiner 150 ppm, ganz besonders bevorzugt kleiner 100 ppm, optimalerweise kleiner 80 ppm und in äußerst optimaler Ausführung kleiner 20 ppm. Dabei sind insbesondere Gehalte kleiner 100 ppm besonders geeignet, Methacrylatharze ohne sichtbare Gelbfärbung herzustellen.

Weiterhin bevorzugt weist das Alkylmethacrylat zusätzlich einen Methylisobutyrat-Gehalt kleiner 600 ppm, besonders bevorzugt kleiner 300 ppm und insbesondere bevorzugt kleiner 100 ppm auf. Neben diesen Alkylmethacrylaten sind ferner Alkylmethacrylatharze, die aus einer Monomermischung, enthaltend 30 bis 100 Gew% der erfindungsgemäßen Alkylmethacrylate hergestellt werden, beschrieben. Diese sind jedoch nicht Bestandteil der vorliegenden Erfindung. Diese Alkylmethacrylatharze können darüber hinaus aus Mischungen, die 0 bis 70 Gew% weiterer mit Alkylmethacrylaten co-polymerisierbarer Monomere und/oder mittels anderer Verfahren hergestellter Alkylmethacrylate, sowie optional 0 bis 5 Gew% weiterer Zuschlagstoffe enthalten, hergestellt werden.

Bevorzugt sind diese Alkylmethacrylatharze dadurch gekennzeichnet, dass dieses Alkylmethacrylatharz ein gewichtsmittleres Molekulargewicht, bestimmt mittels Gel Permeations Chromatography (GPC), zwischen 50.000 g/mol und 2.000.000 g/mol aufweist. Insbesondere bevorzugt handelt es sich bei dem in dem Alkylmethacrylatharz verwendeten, erfindungsgemäß herstellbaren Alkylmethacrylat um Methylmethacrylat. Der überraschend identifizierte negative Effekt, den das DMIB in einem Alkylmethacrylatharz und den daraus hergestellten Folgeprodukten bewirkt, ist neben der Gelbfärbung eine verringerte thermische Stabilität des Produkts. Dies ist auf einen stärkeren Polymerkettenabbau bei der thermischen Verarbeitung zurückzuführen und kommt insbesondere bei der Verarbeitung zu einem Formkörper und bei der Aufarbeitung des Polymersirups zum Tragen. Überraschend wurde jetzt festgestellt, dass ein erfindungsgemäß hergestelltes Methacrylatharz beide Nachteile nicht aufweist.

### Beispiele

Um die Qualität der Alkylmethylacrylatharze zu untersuchen, wurde Methylmethacylat gemäß den Verfahrensschritten a bis d aus dem erfindungsgemäßen Verfahren zur Herstellung von PMMA polymerisiert. Aus dem erhaltenen Polymer wurden anschließend 145 mm lange Messformkörpern hergestellt, an denen die optischen Eigenschaften gemessen wurden.

Für die Herstellung der Polymere wurden folgende Rohstoffe eingesetzt:
Methylmethacrylat von Evonik Industries und aus Verfahren a bis d stabilisiert mit 3 ppm Hydroquinonmonomethylether.
n-Dodecylmercaptan wurde von der Firma Chevron Phillips und
tert-Butylperisononanoat von der Firma United Initiator GmbH bezogen.

Zur Polymerisation wurden die Edukte kontinuierlich einem kontinuierlich betriebenen Rührkessel mit einem Innenvolumen von 2,4 L zugeführt und dabei darauf geachtet, dass die Polymerisationstemperatur immer im Bereich zwischen 120 °C und 150 °C liegt. Die Polymerisation erfolgte bis zu einem Monomer-Umsatz von 55%. Die Restmonomere des Polymersirups des Auslaufes wurden auf einem Extruder bei 250°C kontinuierlich entgast. Die so erhaltenen Polymerstränge der entgasten Polymerschmelze wurden an der Luft abgekühlt und anschließend granuliert.

Reaktorzulauf für die Polymerisation
3500 g/h Methylmethacrylat
7,0 g/h n-Dodecylmercaptan
2,0 g/h tert-Butylperisononanoat

Zur Beurteilung der optischen Qualität der Polymere wurde Polymergranulat bei 220 °C und 50 bar Druck zu Formkörpern gepresst, aus denen dann Stäbe mit den Abmessungen 10 mm x 10 mm x 145 mm herausgeschnitten und die Oberflächen mittels Diamantschleifer poliert wurde.

Der Gelbwert Y.I. und Transmissionskoeffizient D65/10° dieses Formkörpers wurden über die 145 mm Länge in einem Varian Cary 5000 gemessen.

Zu den spezifischen Beispielen sind im Weiteren die jeweiligen Nebenproduktanteile an DMIB bzw. Methylisobutyrat in den Monomeren aus Verfahrensschritt d angegeben. Die in den Beispielen 3 und 4 eingesetzten MMA-Chargen dienen als Referenz. Dieses jeweilige MMA wurde mittels eines C3-Verfahrens produziert und enthält entsprechend weder DMIB, noch Methylisobutyrat.

### Beispiel 1

Methylmethacrylat aus Verfahren a bis d mit
<6 ppm DMIB; 230 ppm Methylisobutyrat
(nach Verfahrensschritt d)

Dabei wurde insbesondere in Bezug auf Verfahrensschritt c folgender Prozess eingesetzt:
Der Austrag aus Verfahrensschritt b, Reaktor II, wurde zur Aufarbeitung in den Verfahrensschritt c, Reaktor III geleitet. Reaktor III wurde als kontinuierlich betriebener Rührkessel mit nachgeschaltetem Dekanter ausgeführt.
pH-Wert in Rührkessel: 2
Verweilzeit in Rührkessel: 60min
Temperatur im Rührkessel: 25 °C
Verweilzeit im Dekanter: 60min
Temperatur im Dekanter: 25 °C

Zuläufe in den Reaktor III, Verfahrensschritt c:
1. Saure wässrige Phase:
   Schwefelsäure 100% H2SO4= 1,05 g/h
   Wasser= 106,05 g/h
2. Zulaufs aus Verfahrensschritt b:
   MMA= 56,11 Gew%
   Methanol= 13,67 Gew%
   DMIB =1659 ppm
   Methylisobutyrat=305 ppm
   Organischer Rest= 18,57 Gew%
   H2O= 11,44 Gew%
   Total Flow : 150 g/h

Zusammensetzung des roh-Alkylmethacrylats nach Verfahrensschritt c:
DMIB < 6ppm (1 ppm in der organischen Phase)
Methylisobutyrat organische Phase = 449 ppm
Methylisobutyrat wässrige Phase= 14 ppm

### Beispiel 2 (Vergleichsbeispiel)

Methylmethacrylat aus Verfahren a, b und d, ohne Verfahrensschritt c mit
1550 ppm DMIB; 475 ppm Methylisobutyrat

### Beispiel 3 (Referenzbeispiel)

Methylmethacrylat von Evonik Industries versetzt mit
1000 ppm DMIB; 50 ppm Methylisobutyrat

### Beispiel 4 (Referenzbeispiel)

Methylmethacrylat von Evonik Industries als Referenz mit
< 5 ppm DMIB; 50 ppm Methylisobutyrat

Im Beispiel 1 wurde MMA gemäß der Verfahrensschritte 1 a bis d eingesetzt und ein geringer Gelbwert und eine hohe Transmission erzielt. Der Gehalt an DMIB ist mit kleiner 6ppm niedrig. Beispiel 2 (Vergleichsbeispiel) verwendet MMA gemäß Verfahrensschritte a, b und d, aber ohne den Aufarbeitungsschritt c. Der Gehalt an DMIB ist mit 1550 ppm höher als im Beispiel 1 wodurch sich ein höherer Gelbwert und eine geringere Transmission des Polymethacrylatharzes ergeben.

In Beispiel 3 und 4 (Referenzbeispiele) wurde jeweils Evonik MMA aus dem ACH Verfahren, in dem kein DMIB gebildet wird, verwendet. Einmal mit künstlich zugesetzten 1000 ppm DMIB und in Beispiel 4 ohne Zusatz von DMIB. Beispiel 3 zeigt wieder einen höheren Gelbwert und eine geringere Transmission als Beispiel 4.

Die Kondensate der entgasten Restmonomere waren bei hohen DMIB-Gehalten im eingesetzten MMA sehr gelb, Beispiel 2 und Beispiel 3.

**Tabelle 1: gegenübergestellte Ergebnisse**

| | MMA | DMIB [ppm] | Methylisobutyrat [ppm] | Y.I. 145mm Polymer [-] | Transmission D65/10° Polymer [%] | Y.I. 10mm Kondensat [-] |
|---|---|---|---|---|---|---|
| Beispiel 1 | Verfahren a-d | <6 | 230 | 4,1 | 92,2 | 3,2 |
| Beispiel 2 | Verfahren a-d / ohne c | 1550 | 475 | 12,0 | 91,5 | 9,8 |
| Beispiel 3 | Evonik Ind. | 1000 | 50 | 7,0 | 91,8 | 8,7 |
| Beispiel 4 | Evonik Ind. | <1 | 50 | 4,1 | 92,7 | 3,5 |

Die jeweiligen Gelbwerte der Kondensate der entgasten Polymersirups werden durch die DMIB Konzentration im eingesetzten Alkylmethacrylat beeinflusst. So sind die Gelbwerte der Vakuumkondensate aus den Beispielen 1 und 4 sehr gering während die Vakuumkondensate der Beispiele 2 und 3 deutlich höher sind.

## Patentansprüche

1. Verfahren zur Herstellung von Alkylmethacrylatharzen, aufweisend die Verfahrensschritte
a. Herstellung von Methacrolein in einem Reaktor I,
b. oxidative Veresterung des Methacroleins in Anwesenheit eine Alkohols, Sauerstoffs und eines heterogenen Edelmetall-haltigen Katalysators bei einem Wassergehalt zwischen 0,1 und 10 Gew% und einem pH-Wert zwischen 5 und 8 in mindestens einem Reaktor II,
c. Nachbearbeitung des Reaktoraustrags aus Reaktor II in einem Reaktor III,
d. Isolierung und Reinigung des roh-Alkylmethacrylats aus Reaktor III und
e. Polymerisation der Alkylmethacrylate aus Verfahrensschritt d oder einer Mischung enthaltend Alkylmethacrylate aus Verfahrensschritt d zum Erhalt eines Alkylmethacrylatharzes,
**dadurch gekennzeichnet, dass** in Verfahrensschritt c in Reaktor III der Wassergehalt um mindestens 0,5 Gew% höher ist als in Reaktor II, die Alkoholkonzentration geringer ist als in Reaktor II und der pH-Wert zwischen 0,5 und 6, dabei mindestens 0,5 geringer als in Reaktor II eingestellt wird.

2. Verfahren gemäß Anspruch 1, **dadurch gekennzeichnet, dass** es sich bei Verfahrensschritt a in Reaktor I um die Umsetzung von Propanal mit Formaldehyd in Gegenwart mindestens einer Säure und optional eines Amins handelt.

3. Verfahren gemäß Anspruch 1 oder 2, **dadurch gekennzeichnet, dass** es sich bei dem Alkohol um Methanol und bei dem Alkylmethacrylat um MMA handelt.

4. Verfahren gemäß mindestens einem der Ansprüche 1 bis 3, **dadurch gekennzeichnet, dass** in Verfahrensschritt c dem Reaktor zur Einstellung des pH-Werts eine organische und/oder eine mineralische Säure zugegeben wird, und dass in diesem Reaktor Dimethoxyisobuten mit Wasser in Methacrolein und Methanol gespalten wird.

5. Verfahren gemäß mindestens einem der Ansprüche 1 bis 4, **dadurch gekennzeichnet, dass** in Verfahrensschritt e aus dem Alkylmethacrylat oder aus einer Mischung enthaltend mindestens ein Alkylmethacrylat aus Verfahrensschritt d mittels Substanz-, Emulsions-, Suspensions- oder Lösungspolymerisation ein Alkylmethacrylatharz hergestellt und anschließend zu einer Formmasse oder in Mischung mit anderen Komponenten zu einer Formmasse verarbeitet wird.

6. Verfahren gemäß mindestens einem der Ansprüche 1 bis 5, **dadurch gekennzeichnet, dass** in Verfahrensschritt e aus dem Alkylmethacrylat aus Verfahrensschritt d oder aus einer Mischung enthaltend zumindest ein Alkylmethacrylat gemäß Verfahrensschritt d mittels radikalischer Polymerisation bis zu einem Feststoffgehalt von weniger als 80% ein Sirup gebildet wird, dieser in eine Form gegossen und dort auspolymerisiert wird.

7. Verfahren gemäß mindestens einem der Ansprüche 1 bis 5, **dadurch gekennzeichnet, dass** in Verfahrensschritt e aus dem Alkylmethacrylat aus Verfahrensschritt d oder aus einer Mischung enthaltend zumindest ein Alkylmethacrylat gemäß Verfahrensschritt d mittels radikalischer Lösungsoder Substanzpolymerisation ein Sirup gebildet wird, dieser optional entgast, optional granuliert, und zu einem Formkörper verarbeitet wird.

8. Verfahren gemäß mindestens einem der Ansprüche 1 bis 5, **dadurch gekennzeichnet, dass** in Verfahrensschritt e aus dem Alkylmethacrylat aus Verfahrensschritt d oder aus einer Mischung enthaltend zumindest ein Alkylmethacrylat gemäß Verfahrensschritt d mittels radikalischer Suspensionspolymerisation bei einem Umsatz von mindestens 80% der Monomere ein Polymerharz gebildet wird, dieses optional getrocknet wird, dieses optional entgast, optional granuliert wird und aus dem Granulat oder der Polymerharzperlen Formkörper hergestellt werden.

9. Verfahren gemäß mindestens einem der Ansprüche 6 bis 8, **dadurch gekennzeichnet, dass** das in einem Entgasungsschritt gewonnene Recyclat zurückgeführt und der Monomermischung in einem Verfahrensschritt e zugeführt wird.

10. Verfahren gemäß mindestens einem der Ansprüche 4 bis 9, **dadurch gekennzeichnet, dass** in Verfahrensschritt c Schwefelsäure zur Einstellung des pH-Werts in Reaktor III gegeben wird, und dass die Flüssigphase in Reaktor III eine Temperatur zwischen 0 und 140 °C aufweist.

11. Verfahren gemäß mindestens einem der Ansprüche 1 bis 10, **dadurch gekennzeichnet, dass** es sich bei Reaktor III um eine Destillationskolonne handelt, in deren Sumpf die Säure und optional zusätzliches Wasser eingeleitet werden, und in der Methacrolein und Teile des verbleibenden Alkohols von dem roh-Alkylmethacrylat bei einer Temperatur zwischen 50 und 100 °C getrennt werden.

12. Verfahren gemäß mindestens einem der Ansprüche 1 bis 10, **dadurch gekennzeichnet, dass** es sich bei Reaktor III um einen Phasentrenner handelt, in dessen wässrige Phase die Säure und optional zusätzliches Wasser eingeleitet werden, und in dem eine wässrige Phase, enthaltend den verbleibenden Alkohol, von einer organischen Phase, enthaltend das Alkylmethacrylat, bei einer Temperatur zwischen 0 und 100 °C getrennt wird.

13. Verfahren gemäß mindestens einem der Ansprüche 1 bis 10, **dadurch gekennzeichnet, dass** es sich bei Reaktor III um einen Rohrreaktor handelt, in dem der Reaktoraustrag aus Reaktor II mit einer Innentemperatur zwischen 50 und 140 °C, die Säure und optional zusätzliches Wasser vermischt werden und diese Mischung anschließend in eine Destillationskolonne oder einen Phasentrenner geleitet wird.

14. Verfahren gemäß mindestens einem der Ansprüche 1 bis 10, **dadurch gekennzeichnet, dass** es sich bei Reaktor III um einen kontinuierlich betriebenen Rührreaktor handelt, in dem der Reaktoraustrag aus Reaktor II mit einer Innentemperatur zwischen 50 und 140 °C, die Säure und optional zusätzliches Wasser vermischt werden und diese Mischung anschließend in eine Destillationskolonne oder einen Phasentrenner geleitet wird.

15. Verfahren gemäß mindestens einem der Ansprüche 1 bis 14, **dadurch gekennzeichnet, dass** es sich bei den Isolierungs- und Reinigungsstufen um mindestens einen Phasentrenner, mindestens eine Hochsiederkolonne, mindestens eine Niedrigsiederkolonne und optional um mindestens eine Kristallisationskammer handelt, und dass diese Vorrichtungen in Reihe durchlaufen werden.

## Claims

1. Process for producing alkyl methacrylate resins, comprising the process steps of
a. production of methacrolein in a reactor I,
b. oxidative esterification of the methacrolein in the presence of an alcohol, oxygen and a heterogeneous noble metal-containing catalyst at a water content between 0.1% and 10% by weight and a pH between 5 and 8 in at least one reactor II,
c. aftertreatment of the reactor output from reactor II in a reactor III,
d. isolation and purification of the crude alkyl methacrylate from reactor III and
e. polymerization of the alkyl methacrylates from process step d or a mixture containing alkyl methacrylates from process step d to obtain an alkyl methacrylate resin,
**characterized in that** in process step c in reactor III the water content is at least 0.5% by weight higher than in reactor II, the alcohol concentration is lower than in reactor II and the pH is set to between 0.5 and 6 and thus at least 0.5 lower than in reactor II.

2. Process according to Claim 1, **characterized in that** process step a in reactor I is the reaction of propanal with formaldehyde in the presence of at least one acid and optionally of an amine.

3. Process according to Claim 1 or 2, **characterized in that** the alcohol is methanol and the alkyl methacrylate is MMA.

4. Process according to at least one of Claims 1 to 3, **characterized in that** in process step c an organic and/or a mineral acid is added to the reactor to adjust the pH and **in that** in this reactor dimethoxyisobutene is cleaved with water to afford methacrolein and methanol.

5. Process according to at least one of Claims 1 to 4, **characterized in that** in process step e from the alkyl methacrylate or from a mixture containing at least one alkyl methacrylate from process step d by means of bulk, emulsion, suspension or solution polymerization an alkyl methacrylate resin is produced and subsequently processed into a molding material or in admixture with other components processed into a molding material.

6. Process according to at least one of Claims 1 to 5, **characterized in that** in process step e from the alkyl methacrylate from process step d or from a mixture containing at least one alkyl methacrylate according to process step d by means of free-radical polymerization up to a solids content of less than 80% a syrup is formed, said syrup is poured into a mold and subjected to final polymerization therein.

7. Process according to at least one of Claims 1 to 5, **characterized in that** in process step e from the alkyl methacrylate from process step d or from a mixture containing at least one alkyl methacrylate according to process step d by means of free-radical solution or bulk polymerization a syrup is formed, said syrup is optionally degassed, optionally granulated and processed into a molding.

8. Process according to at least one of Claims 1 to 5, **characterized in that** in process step e from the alkyl methacrylate from process step d or from a mixture containing at least one alkyl methacrylate according to process step d by means of free-radical suspension polymerization at a conversion of at least 80% of the monomers a polymer resin is formed, said resin is optionally dried, said resin is optionally degassed, optionally granulated and from the granulate or the polymer resin beads moldings are produced.

9. Process according to at least one of Claims 6 to 8, **characterized in that** the recyclate obtained in a degassing step is recycled and supplied to the monomer mixture in a process step e.

10. Process according to at least one of Claims 4 to 9, **characterized in that** in process step c sulfuric acid is added to reactor III to adjust the pH and **in that** the liquid phase in reactor III has a temperature between 0°C and 140°C.

11. Process according to at least one of Claims 1 to 10, **characterized in that** reactor III is a distillation column into the bottom of which the acid and optionally additional water are introduced and in which methacrolein and portions of the remaining alcohol are separated from the crude alkyl methacrylate at a temperature between 50°C and 100°C.

12. Process according to at least one of Claims 1 to 10, **characterized in that** reactor III is a phase separator into whose aqueous phase the acid and optionally additional water are introduced and in which an aqueous phase containing the remaining alcohol is separated from an organic phase containing the alkyl methacrylate at a temperature between 0°C and 100°C.

13. Process according to at least one of Claims 1 to 10, **characterized in that** reactor III is a tubular reactor in which the reactor output from reactor II having an internal temperature between 50°C and 140°C, the acid and optionally additional water are mixed and this mixture is subsequently passed into a distillation column or a phase separator.

14. Process according to at least one of Claims 1 to 10, **characterized in that** reactor III is a continuously operated stirred reactor in which the reactor output from reactor II having an internal temperature between 50°C and 140°C, the acid and optionally additional water are mixed and this mixture is subsequently passed into a distillation column or a phase separator.

15. Process according to at least one of Claims 1 to 14, **characterized in that** the isolation and purification stages are at least one phase separator, at least one high boiler column, at least one low boiler column and optionally at least one crystallization chamber and **in that** these apparatuses are traversed in series.

## Revendications

1. Procédé pour la préparation de résines de méthacrylate d'alkyle, présentant les étapes de procédé :
a. préparation de méthacroléine dans un réacteur I,
b. estérification par oxydation de la méthacroléine en présence d'un alcool, d'oxygène et d'un catalyseur hétérogène contenant un métal noble à une teneur en eau entre 0,1 et 10% en poids et à un pH entre 5 et 8 dans au moins un réacteur II,
c. post-traitement du produit évacué du réacteur II dans un réacteur III,
d. isolement et purification du méthacrylate d'alkyle brut du réacteur III et
e. polymérisation du méthacrylate d'alkyle de l'étape de procédé d ou d'un mélange contenant des méthacrylates d'alkyle de l'étape de procédé d pour obtenir une résine de méthacrylate d'alkyle,
**caractérisé en ce que**, dans l'étape de procédé c dans le réacteur III, la teneur en eau est supérieure d'au moins 0,5% en poids à celle dans le réacteur II, la concentration en alcool est inférieure à celle dans le réacteur II et le pH est réglé entre 0,5 et 6 et ainsi au moins 0,5 en-dessous de celui dans le réacteur II.

2. Procédé selon la revendication 1, **caractérisé en ce qu'**il s'agit, pour l'étape de procédé a dans le réacteur I, de la transformation de propanal avec du formaldéhyde en présence d'au moins un acide et éventuellement d'une amine.

3. Procédé selon la revendication 1 ou 2, **caractérisé en ce que** pour l'alcool, il s'agit du méthanol et, pour le méthacrylate d'alkyle, de MMA.

4. Procédé selon au moins l'une quelconque des revendications 1 à 3, **caractérisé en ce que**, dans l'étape de procédé c, un acide organique et/ou un acide minéral est ajouté au réacteur pour le réglage du pH et **en ce que**, dans ce réacteur, du diméthoxyisobutène est dissocié avec de l'eau en méthacroléine et en méthanol.

5. Procédé selon au moins l'une quelconque des revendications 1 à 4, **caractérisé en ce que**, dans l'étape de procédé e, une résine de méthacrylate d'alkyle est préparée à partir du méthacrylate d'alkyle ou à partir d'un mélange contenant au moins un méthacrylate d'alkyle provenant de l'étape de procédé d par polymérisation en masse, en émulsion, en suspension ou en solution et ladite résine est ensuite transformée en une masse de moulage ou elle est transformée en mélange avec d'autres composants en une masse de moulage.

6. Procédé selon au moins l'une quelconque des revendications 1 à 5, **caractérisé en ce que**, dans l'étape de procédé e, on forme à partir du méthacrylate d'alkyle provenant de l'étape de procédé d ou à partir d'un mélange contenant au moins un méthacrylate d'alkyle selon l'étape de procédé d, par polymérisation radicalaire jusqu'à une teneur en solides inférieure à 80%, un sirop, celui-ci est versé dans un moule et sa polymérisation est terminée dans celui-ci.

7. Procédé selon au moins l'une quelconque des revendications 1 à 5, **caractérisé en ce que**, dans l'étape de procédé e, on forme à partir du méthacrylate d'alkyle provenant de l'étape de procédé d ou à partir d'un mélange contenant au moins un méthacrylate d'alkyle selon l'étape de procédé d, par polymérisation radicalaire en solution ou en masse, un sirop, celui-ci est éventuellement dégazé, éventuellement granulé et transformé en un corps moulé.

8. Procédé selon au moins l'une quelconque des revendications 1 à 5, **caractérisé en ce que**, dans l'étape de procédé e, on forme à partir du méthacrylate d'alkyle provenant de l'étape de procédé d ou à partir d'un mélange contenant au moins un méthacrylate d'alkyle selon l'étape de procédé d, par polymérisation radicalaire en suspension à une conversion d'au moins 80% des monomères, une résine polymère, celle-ci est éventuellement séchée, éventuellement dégazée, éventuellement granulée, éventuellement transformée et des corps moulés sont formés à partir du granulat ou à partir des billes de résine polymère.

9. Procédé selon au moins l'une quelconque des revendications 6 à 8, **caractérisé en ce que** le produit de recyclage obtenu dans une étape de dégazage est recyclé et introduit dans le mélange de monomère dans une étape de procédé e.

10. Procédé selon au moins l'une quelconque des revendications 4 à 9, **caractérisé en ce que**, dans l'étape de procédé c, pour le réglage du pH, de l'acide sulfurique est introduit dans le réacteur III et **en ce que** la phase liquide dans le réacteur III présente une température entre 0 et 140°C.

11. Procédé selon au moins l'une quelconque des revendications 1 à 10, **caractérisé en ce qu'**il s'agit, pour le réacteur III, d'une colonne de distillation dans le fond de laquelle sont introduits l'acide et éventuellement de l'eau supplémentaire et dans laquelle la méthacroléine et des parties de l'alcool résiduel du méthacrylate d'alkyle brut sont séparées à une température entre 50 et 100°C.

12. Procédé selon au moins l'une quelconque des revendications 1 à 10, **caractérisé en ce qu'**il s'agit, pour le réacteur III, d'un séparateur de phase, dans la phase aqueuse duquel sont introduits l'acide et éventuellement de l'eau supplémentaire et dans lequel une phase aqueuse, contenant l'alcool résiduel, est séparée d'une phase organique contenant le méthacrylate d'alkyle à une température entre 0 et 100°C.

13. Procédé selon au moins l'une quelconque des revendications 1 à 10, **caractérisé en ce qu'**il s'agit, pour le réacteur III, d'un réacteur tubulaire dans lequel sont mélangés le produit évacué du réacteur II à une température interne entre 50 et 140°C, l'acide et éventuellement de l'eau supplémentaire et ce mélange est ensuite guidé dans une colonne de distillation ou un séparateur de phases.

14. Procédé selon au moins l'une quelconque des revendications 1 à 10, **caractérisé en ce qu'**il s'agit, pour le réacteur III, d'un réacteur tubulaire exploité en continu, dans lequel sont mélangés le produit évacué du réacteur II à une température interne entre 50 et 140°C, l'acide et éventuellement de l'eau supplémentaire et ce mélange est ensuite guidé dans une colonne de distillation ou un séparateur de phases.

15. Procédé selon au moins l'une quelconque des revendications 1 à 14, **caractérisé en ce qu'**il s'agit, pour les étages d'isolement et de purification, d'au moins un séparateur de phase, d'au moins une colonne à point d'ébullition élevé, d'au moins une colonne à bas point d'ébullition et éventuellement d'au moins une chambre de cristallisation et **en ce que** ces dispositifs sont traversés en série.
